Europäisches Patentamt

European Patent Office

Office européen des brevets

(11) Veröffentlichungsnummer: **0 185 612**
**A1**

(12) **EUROPÄISCHE PATENTANMELDUNG**

(21) Anmeldenummer: **85810525.7**

(22) Anmeldetag: **11.11.85**

(51) Int. Cl.⁴: **A 01 N 43/20**
**A 61 L 2/20, C 09 K 7/00**

(30) Priorität: **10.12.84 CH 5852/84**

(43) Veröffentlichungstag der Anmeldung:
**25.06.86 Patentblatt 86/26**

(84) Benannte Vertragsstaaten:
**AT BE CH DE FR GB IT LI LU NL SE**

(71) Anmelder: **Sanitized Verwertungs A.-G.**
**Murbacherstrasse 3**
**CH-6003 Luzern(CH)**

(72) Erfinder: **Mebes, Bruno**
**Lyssachstrasse 95**
**CH-3400 Burgdorf(CH)**

(74) Vertreter: **Rochat, Daniel Jean et al,**
**Bovard AG Patentanwälte VSP Optingenstrasse 16**
**CH-3000 Bern 25(CH)**

(54) **Desinfektion von Erdöl und Erdölprodukten und ihre Anwendung bei der Erdölförderung.**

(57) Das Verfahren zur Desinfektion von Erdöl und Erdölprodukten verwendet als Wirkstoff Propylenoxid. Das Verfahren eignet sich besonders zur Hemmung des unerwünschten Wachstums von Mikroorganismen während der Gewinnung von Erdöl.

EP 0 185 612 A1

Croydon Printing Company Ltd

- 1 -

Desinfektion von Erdöl und Erdölprodukten
und ihre Anwendung bei der Erdölförderung

Die vorliegende Erfindung betrifft ein Verfahren zur Desinfektion von Erdölprodukten, sowie die Anwendung dieses Verfahrens bei der Erdölförderung.

Bei der Schürfung von Erdöl wie auch bei seiner Verarbeitung können die einzelnen Arbeitsabläufe durch mikrobielle Kontaminationen gestört werden. Das Wachstum von Mikroorganismen in Oelen erfordert die Gegenwart von Wasser, einer Stickstoffquelle und nötigen Spurenelementen. Diese Wachstumsvoraussetzungen sind bei den meisten in der Ausbeutung befindlichen Erdöllagerstätten vorhanden. Beispielsweise werden bei der Offshore-Gewinnung von Erdöl durch die Bohrung und auch durch die Injektion von grossen Quantitäten von Meerwasser unerwünschte Mikroorganismen in die Oelquelle transferiert. Diese Mikroorganismen, bzw. ihre Metaboliten, können zu Störungen bei der Förderung des Erdöls führen, beispeilsweise Verstopfungen der Leitungen, Gasbildung oder Korrosion von Installationen.

Zur Hemmung des Wachstums solcher Mikroorganismen werden Biozide, wie Glutaraldehyd, Acrolein oder quartäre Ammoniumverbindungen (Quats) eingesetzt. Diese Desinfektionsmittel zeigen aber bestimmte Nachteile wie Substantivität zur Oberfläche oder Verharzungen. Geeignete Biozide für die Anwendung bei der Erdölgewinnung müssen ökologisch unbedenklich sein, ein weites mikrobielles Spektrum abdecken, in kleinen Konzentrationen wirksam sein, nicht selektiv wirken, und bei den Anwendungstemperaturen eine angemessene Stabilität aufweisen. Weiter sollten die Biozide keine Korrosion verursachen, leicht zu handhaben und billig sein. Die bekannten Mittel können zu unerwünschten Strömungsverlusten, zu Sedimentationen und sogar Blockierungen führen, was den Arbeitsablauf in schwerwiegender Weise stören kann.

Es ist somit Aufgabe der vorliegenden Erfindung, ein Desinfektionsverfahren zur Verfügung zu stellen, durch welches die Mikroorganismen ohne die obengenannten Nachteile wirkungsvoll bekämpft werden können.

Gegenstand der vorliegenden Erfindung ist somit ein Verfahren zur Desinfektion von Erdöl und Erdölprodukten, worin dem Rohöl oder den Erdölprodukten eine mikrobiozide Menge Propylenoxid zugesetzt wird, und seine Anwendung bei der Gewinnung von Erdöl.

Im erfindungsgemässen Desinfektionsverfahren wird das Propylenoxid in der Regel in einer zweckmässigen Konzentration mit einem inerten Träger vermischt, sodass eine sichere Handhabung des Mittels gewährleistet ist.

Propylenoxid und die bevorzugten Trägermaterialien erlauben durch ihre Verträglichkeit mit den in der Erdölindustrie verwendeten Chemikalien und dem Erdöl selbst eine einwandfreie Dosierung und Applikation in sämtlichen Verarbeitungsstufen.

Die Anwendung des erfindungsgemässen Desinfektionsverfahrens ist nicht allein auf die Erdölgewinnung beschränkt, sondern kann ebenfalls für weitere Verfahrensstufen und die Lagerung von Erdölprodukten angewendet werden. Insbesondere die Gegenwart von Wasser macht Erdöl und die Erdölprodukte anfällig auf Mikroorganismen. Das erfindungsgemässe Verfahren ist deshalb besonders für Erdöl und Erdölprodukte geeignet, in deren Gegenwart sich ebenfalls Wasser befindet. So ist es beispielsweise möglich, dass bei der Komplettierung das Propylenoxid durch das Wasser in die Erdölquelle transportiert wird. Dadurch werden im Wasser befindliche Mikroorganismen bereits während diesem Transportvorgang bekämpft und können bei Aufrechterhaltung einer minimalen Konzentration Propylenoxid das Erdöl nicht kontaminieren. Durch den Kontakt des Propylenoxid enthaltenden Wassers mit dem Erdöl geht ein Teil des Propylenoxides in das Erdöl über, wo es ebenfalls seine biozide Aktivität entfalten kann.

Vorzugsweise wird das Propylenoxid in einer solchen Menge appliziert, dass das zu desinfizierende System eine Propylenoxidkonzentration von 15 - 20 ppm aufweist. Geeignete Trägermaterialien zur Applikation sind Leichtparaffinöl, Polyetherverbindungen, wie Nonylphenolpolyglykolether mit 10 Ethylenoxideinheiten oder Fettalkoholpolyglykolether mit 12 - 16 C-Atomen im Alkoholteil und 10 - 12 Ethylenoxideinheiten, Wasser oder Mischungen davon. Weiter sind auch anionenaktive, kationenaktive und amphotere Emulsionssysteme geeignet.

Die nachstehenden Beispiele stellen bevorzugte Ausführungsformen von Formulierungen von Propylenoxid dar, wie sie im erfindungsgemässen Verfahren eingesetzt werden können.

### Beispiel 1

Es wird eine Mischung von folgenden Bestandteilen hergestellt:

15 Gewichtsteile Propylenoxid,
50 Gewichtsteile Leichtparaffinöl, und
35 Gewichtsteile Nonylphenolpolyglykolether
        mit 10 Ethylenoxideinheiten.

Diese Mischung eignet sich für den Einsatz in Wasser-in-Oel- oder Oel-in-Wasser-Systemen. Es wird eine wirksame Desinfektion der Systeme erzielt, wenn die Propylenoxidkonzentration 5 - 20 ppm beträgt.

Beispiel 2
Es werden folgende Bestandteile gemischt:
20 Gewichtsteile Propylenoxid,
40 Gewichtsteile White Spirit, und
40 Gewichtsteile Fettalkoholpolyglykolether
mit 12 - 16 C-Atomen im Alkoholteil und
10 - 12 Ethylenoxideinheiten.

Diese Formulierung wird zur Desinfektion einer Erdöl/Wassermischung in einer solchen Konzentration zugegeben, dass eine Propylenoxidkonzentration von 5 - 20 ppm erzielt wird.

Das erhaltene Gemisch wird in einem Druckdosierpumpensystem in ein zu behandelndes Rohrsystem eingegeben. Die Fördermenge wird so eingestellt, dass die Konzentration des Propylenoxides im zu desinfizierenden System mindestens 5 - 20 ppm Propylenoxid beträgt. Dabei wird eine einwandfreie Desinfektion erzielt. In gleicher Weise und mit gleicher Wirkung kann ebenfalls Ethylenoxid als desinfizierender Wirkstoff verwendet werden. Auf Grund seiner physikalischen Eigenschaften weist dieser Stoff jedoch gegenüber Propylenoxid Nachteile in der Handhabung auf.

0185612

Die nachstehende Tabelle erläutert das erfindungsgemässe Desinfektionsverfahren an einer
mit Mikroorganismen kontaminierten Oelemulsion.
Als Vergleich dienen einerseits entsprechende mit
Glutaraldehyd behandelte, kontaminierte Oelemulsionen und andererseits unbehandelte kontaminierte
Oelemulsionen.

## Tabelle I

Wirkung von Propylenoxid auf verschiedene Problemkeime der erdölverarbeitenden Industrie im Vergleich mit Glutaraldehyd.

Versuchsbedingungen: Keimzahl / ml Oelemulsion = Log 5

Oelemulsion: 15 % Crudeoil, 75 % Wasser mit Emulgator

Kontaktzeit: 15 bzw. 30 min

Desinfektionsmittel: 20 ppm Glutaraldehyd, bzw. 20 ppm Propylenoxyd

Temperatur: 25 °C

| Keimart: | Kontaktzeit in min 15 min ohne | mit | 30 min ohne | mit | Desinfektionsmittel bzw. Applikation |
|---|---|---|---|---|---|
| Pseudomonas aeruginosa ATCC 15442 | ++++ | ---- | ++++ | ---- | Formulierung und Applikation nach Beispiel 1 |
| Pseudomonas aeruginosa ATCC 15442 | ++++ | +(+) | ++++ | + | Glutaraldehyd |
| E.Coli ATCC 11229 | ++++ | (+)(+) | ++++ | ---- | Formulierung und Applikation nach Beispiel 1 |
| E.Coli ATCC 11229 | ++++ | ++ | ++++ | (+)(+) | Glutaraldehyd |
| Proteus vulgaris ATCC 6896 | ++++ | + | ++++ | ---- | Formulierung und Applikation nach Beispiel 1 |

| Keimart: | Kontaktzeit in min 15 min | | 30 min | | Desinfektionsmittel bzw. Applikation |
|---|---|---|---|---|---|
| | ohne | mit | ohne | mit | |
| Proteus vulgaris ATCC 6896 | ++++ | ++ | ++++ | (+)(+) | Glutaraldehyd |
| Staph.aureus ATCC 6538 | ++++ | ---- | ++++ | ---- | Formulierung und Applikation nach Beispiel 1 |
| Staph.aureus ATCC 6538 | ++++ | (+) | ++++ | ---- | Glutaraldehyd |
| Bact.subtilis ATCC 6633 | ++++ | + | ++++ | ---- | Formulierung und Applikation nach Beispiel 1 |
| Bact.subtilis ATCC 6633 | ++++ | +++ | ++++ | + | Glutaraldehyd |
| Desulfovibrio salexigenes ATCC 14822 NCIB 8403 | ++++ | ---- | ++++ | ---- | Formulierung und Applikation nach Beispiel 1 |
| Desulfovibrio salexigenes ATCC 14822 NCIB 8403 | ++++ | +++ | ++++ | ++ | Glutaraldehyd |
| Sphaerotilus natans | ++++ | ---- | ++++ | ---- | Formulierung und Applikation nach Beispiel 2 |
| Sphaerotilus natans | ++++ | ++ | ++++ | + | Glutaraldehyd |

- 7 -

0185612

| Keimart: | Kontaktzeit in min 15 min ohne | mit | 30 min ohne | mit | Desinfektionsmittel bzw. Applikation |
|---|---|---|---|---|---|
| Saccharomyces cerevisiae | ++++ | ++ | ++++ | ---- | Formulierung und Applikation nach Beispiel 2 |
| Saccharomyces cerevisiae | ++++ | ++ | ++++ | + | Glutaraldehyd |
| Aspergillus niger ATCC 6275 | ++++ | (+) | ++++ | ---- | Formulierung und Applikation nach Beispiel 2 |
| Aspergillus niger ATCC 6575 | ++++ | +++ | ++++ | ++ | Glutaraldehyd |
| Penicillium funiculosum IAM 7013 | ++++ | ---- | ++++ | ---- | Formulierung und Applikation nach Beispiel 2 |
| Penicillium funiculosum IAM 7013 | ++++ | + | ++++ | ---- | Glutaraldehyd |

Bemerkung: Legende: ++++ = ungehemmtes Wachstum
+++ = Wachstumshemmung ca. 25 %
++ = Wachstumshemmung ca. 50 %
+ = Wachstumshemmung ca. 75 %
(+)= sehr schwaches Wachstum
- = kein Wachstum

Das erfindungsgemäss eingesetzte Biozid im
Vergleich mit bekannten Bioziden, welche bei
der Erdölförderung eingesetzt werden.

Die nachstehenden Biozide finden zur Desinfektion von Wasserkreislaufsystemen für die Erdölförderung (z.B. Drilling-fluids) Anwendung. Momentan findet nur eine beschränkte Anzahl Chemikalien auf dem Gebiet der Erdölförderung bzw. Verarbeitung Anwendung.

Die bekanntesten sind:

| Biozid | Konzentration in ppm |
|---|---|
| Isothiazolin | 15 - 30 |
| Glutaraldehyd | 25 - 100 |
| aliphatische Amine | 50 - 110 |
| quaternäre Ammoniumverbindungen | 50 - 100 |
| Acrolein | 5 - 15 |
| Dibrommononitrilopriprionamid | 30 - 80 |
| Propylenoxid (erfindungsgemäss) | 5 - 10 |

In einer Arbeit von Ruseska, Robbins und Costerton, Oil & Gas Journal, March 8, 1982 wurde nachgewiesen, dass geeignete wirksame Biozide die Fähigkeit besitzen müssen, in Biofilme einzudringen, um Mikroorganismen zu zerstören. Die Rohre sind in der Regel von der GLYCOCALYX, einem anionischen Polysacharidfilm bedeckt und die Mikroorganismen sind in diesen Film eingebettet. D.h. die aktive biozide Komponente muss in polaren und unpolaren Systemen löslich sein. Ein Wirkstoff, welcher nur in Wasser löslich ist, hat nur einen Einfluss auf die Wachstumsrate von nicht-sessilen Bakterien, ist jedoch für die Bekämpfung von sessilen, Bakterien, wie sulfatreduzierende Bakterien (SRB-Typ), wirkungslos.

Die Ergebnisse eines Screening-Tests sind in der nachstehenden Tabelle angeführt.

- 10 -

Die Tests wurden mit einer Robbins-Device durchgeführt, welche in McCoy, W.I., Bryers, J.D., Robbins, J., and Costerton, J.W., "Observations on biofilm formation". Canadian Journal of Microbiology, Vol.27, pp. 910-917, 1981 beschrieben ist.

Tabelle II     Bakterienwachstum unter aeroben und anaeroben Bedingungen, nichtstationäre Phase $cbu/cm^{2*}$ in log-Einheit

| im Test verwendete Biozide | ppm | Aerobe, sessile Bakterien cbu/cm² Pseudomonas aeruginosa ATCC 15442 | | | | Sulfat reduzierende sessile Bakterien cbu/cm² SRB wilder Stamm | | | | Glycocalys Film | | |
|---|---|---|---|---|---|---|---|---|---|---|---|---|
| | | 6 h | 12 h | 24 h | 48 h | | | | | | | |
| Glutaraldehyd 40% | 25 | log 8,4 | log 8,8 | log 7,2 | log 7,4 | log 8,2 | log 8,7 | log 8,2 | log 7,3 | + | + | ++ |
| Glutaraldehyd 40% | 50 | log 7,8 | log 5,7 | log 5,2 | log 4,8 | log 8,1 | log 7,4 | log 6,9 | log 5,1 | + | + | (+) |
| Glutaraldehyd 40% | 100 | log 6,8 | log 5,1 | log 4,6 | log 3,8 | log 7,2 | log 6,4 | log 4,9 | log 4,6 | - | - | - |
| Acrolein 98 % | 2 | log 7,9 | log 6,8 | log 5,9 | log 4,7 | log 8,1 | log 7,2 | log 6,1 | log 5,1 | + | (+) | - |
| Acrolein 98 % | 5 | log 6,6 | log 5,3 | log 4,6 | log 3,7 | log 7,7 | log 6,4 | log 5,0 | log 4,1 | + | - | - |
| Acrolein 98 % | 10 | log 4,9 | log 3,6 | log 2,1 | < log 2 | log 4,8 | log 3,3 | log 2,3 | log 2 | - | - | - |
| Propylenoxid in White Spirit | 2 | log 7,3 | log 6,7 | log 5,8 | log 4,5 | log 7,9 | log 7,3 | log 5,9 | log 4,9 | + | - | - |
| Propylenoxid in White Spirit | 5 | log 6,2 | < log 2 | < log 2 | < log 2 | log 5,9 | < log 2 | < log 2 | < log 2 | - | - | - |
| Propylenoxid in White Spirit | 10 | < log 2 | < log 2 | < log 2 | < log 2 | log 3,1 | < log 2 | < log 2 | < log 2 | - | - | - |

\* Colony building unit pro cm² entspricht der Keimzahl pro cm²

## Patentansprüche

1.      Verfahren zur Desinfektion von Erdöl und Erdölprodukten, insbesondere zur Hemmung des Wachstums von darin lebensfähigen Mikroorganismen, dadurch gekennzeichnet, dass dem Erdöl oder den Erdölprodukten eine mikrobiozide Menge Propylenoxid zugesetzt wird.

2.      Verfahren nach Anspruch 1, dadurch gekennzeichnet, dass das Propylenoxid zusammen mit einem inerten Träger appliziert wird.

3.      Verfahren nach Anspruch 2, dadurch gekennzeichnet, dass der inerte Träger ein Gemisch von Leichtparaffinöl und Nonylphenolpolyglykolether mit 10 Ethylenoxideinheiten oder ein Gemisch von White Spirit und $C_{12-16}$-Fettalkoholpolyglykolether mit 10-12 Ethylenoxideinheiten enthält.

4.      Verfahren nach Anspruch 2, dadurch gekennzeichnet, dass der inerte Träger ein anionenaktives, kationenaktives oder amphoteres Emulsionssystem ist.

5.      Verfahren nach einem der Ansprüche 1 - 4, dadurch gekennzeichnet, dass die Konzentration des Propylenoxides im Erdöl oder Erdölprodukt 15-20 ppm beträgt.

6.      Verfahren nach einem der Ansprüche 1 - 5, dadurch gekennzeichnet, dass das Erdöl oder die Erdölprodukte in Form einer Oel-in-Wasser- oder Wasser-in-Oel-Suspension vorliegen.

7.    Anwendung des Verfahrens nach einem der Ansprüche 1 - 6 bei der Gewinnung von Erdöl.

8.    Anwendung nach Anspruch 7 bei der Komplettierung des Borhloches.

9.    Anwendung des Verfahrens nach Anspruch 7 auf eine Erdölförderung mittels Wasserinjektion, wobei das Propylenoxidgemisch dem injizierten Wasser zugesetzt wird.

**0185612**

Nummer der Anmeldung

EP 85 81 0525

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Ci 4) |
|---|---|---|---|
| X | CHEMICAL ABSTRACTS, Band 86, Nr. 11, 14. März 1977, Seite 467, Nr. 70669z, Columbus, Ohio, US; A.S. LOPES et al.: "Comparative effects of methylbromide, propylene oxide and autoclave sterilization on specific soil chemical characteristics", & TURRIALBA 1976, 26(4), 351-5 | 1,2,4-9 | A 01 N 43/20 A 61 L 2/20 C 09 K 7/00 |
| Y | Idem | 3 | |
| | --- | | |
| X | CHEMICAL ABSTRACTS, Band 70, Nr. 22, 2. Juni 1969, Seite 235, Nr. 105426s, Columbus, Ohio, US; M.J. NAYLON et al.: "Propylene oxide sterilization of media containing bacterial populations", & PROC. IOWA ACAD. SCI. 1967 (Pub. 1968), 74, 20-5 | 1,2,4-9 | |
| | | | **RECHERCHIERTE SACHGEBIETE (Int. Ci 4)** |
| Y | Idem | 3 | A 01 N A 61 L C 09 K |
| | --- | | |
| Y | EP-A-0 019 470 (UNILEVER) * Seite 2, Zeile 33; Seiten 5,6; Seite 9, Zeile 9 * | 3 | |
| | ----- | | |

Der vorliegende Recherchenbericht wurde für alle Patentansprüche erstellt

| Recherchenort DEN HAAG | Abschlußdatum der Recherche 02-04-1986 | Prüfer PELTRE CHR. |
|---|---|---|